# EUROPEAN PATENT APPLICATION

(11) **EP 1 090 909 A1**
(43) Date of publication of application: **11.04.2001**
(21) Application number: 00203377.7
(22) Date of filing: 28.09.2000
(51) Int. Cl.: C07C 237/12, C07D 233/64, C07F 9/09, A61K 31/165, A61K 31/415

(54) **Novel inhibitors of prenylated pyrophosphate consuming enzymes**

(30) Priority: 28.09.1999 EP 99203170
(71) Applicant: RIJKSUNIVERSITEIT LEIDEN, 2300 RA Leiden (NL); NEDERLANDSE ORGANISATIE VOOR TOEGEPAST-NATUURWETENSCHAPPELIJK ONDERZOEK TNO, 2628 VK Delft (NL)
(72) Inventor: Burm, Brigitte Elisa Anna, 3705 TD (NL); Voskuyl, Nicolette, 2324 Leiden (NL); Van Den Elst, Hans, 2182 TV Hillegom (NL); Pieterman, Elsbet Jantine, 2312 KM Leiden (NL); Cohen, Louis Hartog, 3621 HR Breukelen (NL); Overhand, Mark, 2312 SZ Leiden (NL); Van Der Marel, Gijsbert Arie, 2318 MD Leiden (NL); Van Boom, Jacobus Hubertus, 2343 CR Oegstgeest (NL)
(74) Representative: Jorritsma, Ruurd

(57) **Abstract**

The invention pertains to novel peptide-like FPP-analogues suitable for inhibiting protein:prenyl transferases, such as protein:geranylgeranyl transferase-1, and other prenyl pyrophosphate-consuming enzymes such as squalene synthase and geranylgeranyl pyrophoshate synthase. The analogues comply with the following formula:

A-B-D (1) or A-B-B'-D(2):

A = Ap-(CH₂)₀₋₃-Z¹-(CH₂)₀₋₃-(CHR¹)₀₋₁Y-
B and B' are independently -NR²-(CH₂)₀₋₃-Z²-(CH₂)₀₋₃-CHR³-Y-, or 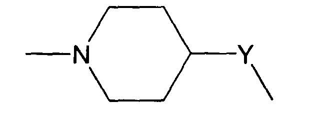
with the proviso that B or B' contains at least 4 chain atoms,
D = -NH-CH(COX)-R⁴
   in which:
Ap = an apolar hydrocarbon group, optionally substituted;
R¹ = hydrogen, C₁₋₄ alkyl optionally substituted with aryl or heteroaryl;
R² = hydrogen, C₁₋₄ alkyl optionally substituted with aryl or heteroaryl;
R³ = hydrogen, C₁₋₄ alkyl optionally substituted with aryl, heteroaryl or amino;
R⁴ = C₁₋₄ alkyl or benzyl, which is substituted with at least one acidic group;
X = OH, OM (M being an alkali metal), O-(C₁₋₄ alkyl), NH-peptidyl or C₁₋₃ alkyl which is substituted with a peptidomimetic group;
Y = carbonyl or methylene;
Z¹ = -CH₂-CHR¹⁰-, -CH=CR¹⁰-, -C≡C-, 1,2-, 1,3- or 1,4-cyclohexylene or -phenylene, -CO-, -CO-NH-, -O-CO-NH or -O-, or a direct bond;
Z²= -CH₂-CHR¹⁰-, -CH=CR¹⁰-, -C≡C-, 1,2-, 1,3- or 1,4-cyclohexylene or -phenylene, -O-, -S-, -NH-, or a direct bond;
or a salt or ester thereof.

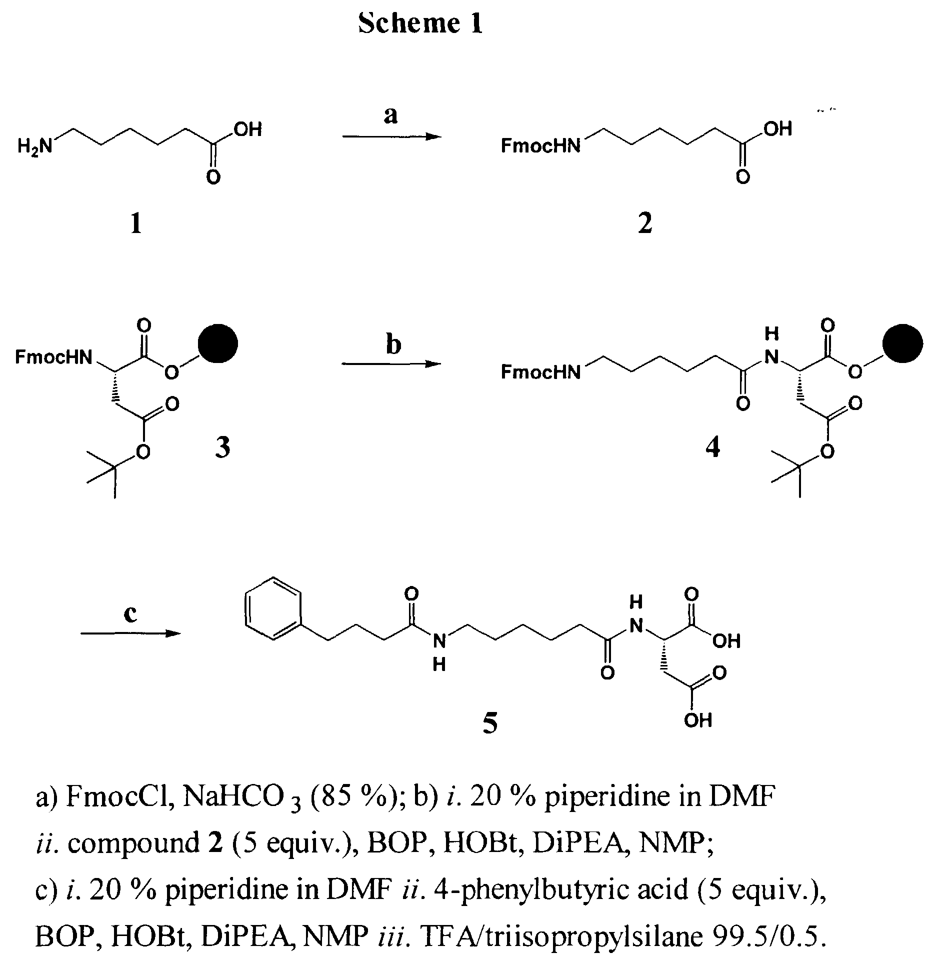

## Description

The present invention relates to peptide analogues which inhibit prenylated pyrophosphate-consuming enzymes. Prenylated pyrophosphate consuming enzymes are understood to comprise protein:farnesyl transferases, protein:geranylgeranyl transferases and several other enzymes involved in the biosynthesis of terpenes, such as farnesyl pyrophosphate synthase, squalene synthase and geranylgeranyl pyrophosphate synthase.

Prenylated pyrophosphates, i.e. *all-trans*-farnesyl pyrophosphate (FPP) and *all*-*trans*-geranylgeranyl pyrophosphate (GGPP), are substrates for a number of different enzymes. Protein:farnesyl transferase (PFT) is an enzyme which uses FPP to catalyse the farnsylation of cysteine residues near the C-terminus of certain proteins. The enzyme geranylgeranyl pyrophosphate synthase (GGPPS) uses FPP as a substrate in the production of geranylgeranyl pyrophosphate (GGPP), which is subsequently used in the synthesis of geranylgeranylated proteins mediated by the enzymes protein:geranylgeranyl transferase 1 and 2 (PGGT-1 and 2).

Several of these prenylated (i.e. farnesylated and/or geranylgeranylated) proteins were identified as belonging to groups of related proteins: e.g. the nuclear lamins, low molecular weight GTP binding proteins (G-proteins), such as the *ras*-oncogene proteins and the γ-subunit of heterotrimeric G-proteins (Schäfer et al., *Science* **245** (1989) 379-385). Lamins and p21^{ras} proteins, 188 or 189 amino acid proteins which possess the consensus CaaX motif (C = cysteine, a = any amino acid having an aliphatic side chain and X = methionine, serine, glutamine or alanine) at the C-terminus, are farnesylated. Other small G-proteins, that have the consensus CaaL motif (L = leucine) such as Rho and several members of the *rab* proteins having C-terminal CC/CXC motifs, arid also heterotrimeric G-protein γ-subunits are geranylgeranylated.

G-proteins are involved in the receptor-mediated transduction of signals (such as growth modulation signals) over the plasma membrane, and other prenylated proteins, not yet identified, may have a function in cell cycle progression. The prenylation of these proteins seems to play a role in their association with membranes and nuclear envelopes, where they are processed further and/or perform their function. This was shown for example by blocking the mevalonate synthesis by HMG-CoA reductase inhibitors, which prevented proteolytic processing of the lamin A precursor (Beck et al., *J. Cell. Biol*. **110** (1990) 1489-1499) or resulted, in other studies, in the accumulation of non-prenylated p21^{ras} precursor and the loss of transforming activity of oncogenic ras proteins. A review of the post-translational modification of proteins by isoprenoids in mammalian cells is given by Maltese W.A. in *FASEB J.* **4** 3319-3329 (1990). The latter observation triggered the search for specific inhibitors of the farnesylation of p21^{ras} in order to prevent its action in cells, where over-expression of this protein leads to tumour development, such as in colon carcinomas.

We have shown that FPP-analogues, which are *in vitro* inhibitors of PFT and/or PGGT-1 are able to inhibit the proliferation of human arterial smooth muscle cells in culture (Cohen et al. *Biochem. Pharm*. **57** (1999), 365-373) and are therefore potential inhibitors of the process of restenosis after percutaneous transluminal coronary angioplasty. The proliferation of smooth muscle cells plays a major role in the latter process.

Very recently it was shown that inhibition of the cellular GGPP synthesis in specific bone cells (osteoclasts) is the possible mechanism of action of anti-osteoporosis drugs (Van Beek, E. *Biochem. Biophys. Res. Commun*. **255**, (1999), 491-494 and Van Beek, E. *J. Bone and Min. Res*. **14** (1999), 722-729). So, inhibitors of GGPPS and/or PGGT-1 and/or -2 may be active as anti-osteoporosis drugs as well.

Furthermore, G-proteins of the rab-family are involved in the regulation of intracellular protein traffic and secretion. In addition, prenylated proteins seem to play a role in the translational control of HMG-CoA reductase, the rate limiting enzyme of the isoprene and subsequent cholesterol biosynthesis. Squalene synthase (SS), the first pathway-specific enzyme in the biosynthesis of cholesterol, catalyses the reductive dimerisation of two molecules of FPP to produce squalene. Much attention has been directed to the development of inhibitors of SS with the aim to lower elevated blood plasma levels of cholesterol, one of the risk factors for cardiovascular diseases.

EP-A-540782 describes inhibitors for protein:farnesyl transferase based on prenyl pyrophosphate analogues. A review of new therapeutic methods based on protein:farnesyl transferase and inhibitors thereof are described by Leonard in *J. Med. Chem*. **40** (1997), 2971-2990. Furthermore, we recently (Overkleeft et al. *Tetrahedron Let*. **40** (1999) 4103-4107) described inhibitors of protein:prenyl transferases based on binding in the CaaX pocket of the enzym concerned (see also EP-A-1028117).

Novel analogues of prenyl pyrophosphate have been found according to the invention, which are inhibitors of prenylated pyrophosphate consuming enzymes (e.g. PFT, PGGT-1 and 2, GGPPS and SS) presumably by binding in the FPP pocket. The analogues are defined in the appending claims with reference to formulae 1 (A-B-D) and 2 (A-B-B'-D). The novel prenylated pyrophosphate-based inhibitors contain unnatural amino acid moieties which have the potential to be unsusceptible towards enzymatic degradation. These novel inhibitors are composed of amino acid derivatives which are ideally suited for the construction of a compound library *via* a combinatorial approach. Furthermore their peptide-like structure allows for easy access to bisubstrate analogues, in which the new FPP analogues are connected to CaaX box analogues, as defined in the appending claims.

Important features of the peptide-like FPP-analogues of the invention are the presence of a hydrophobic region (part A), a spacer moiety comprising one or two (un)natural amino acids (parts B and B 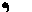 ) and, most preferably, a negatively charged amino acid residue at the C-terminus (part D). The hydrophobic region contains at least six carbon atoms (including further groups such as a carbonyl or oxymethylene group) up to about 24 carbon atoms, preferably between 8 and 18 carbon atoms, and may comprise alkyl, alkenyl, cycloalkyl, cycloalkenyl or aryl groups which may also contain heteroatoms. These groups may contain substituents, which are preferably non-polar, such as C₁-C₄ alkyl, C₁-C₄ alkoxy, and the like. Examples of non-aromatic moieties are hexyl, isooctyl, decyl, dodecyl, cyclopentenyl, cyclohexyl, methylcyclohexyl, tetra-hydropyranyl, and extensions thereof such as cyclohexylmethylcarbonyl, cyclohexylethyl, lauroyl, and the like. Aryl and especially aralkyl groups are preferred. Aromatic moieties include phenyl, tolyl, methoxyphenyl, isopropoxyphenyl, methylenedioxyphenyl, tetrahydronaphthyl, pyridyl, pyrimidyl, furyl, imidazolyl, thiazolyl, naphthyl, phenoxyphenyl, indolyl, anthryl, and the corresponding benzoyl, phenylethyl, cinnamyl and longer aralk(en)yl derivatives. Examples of part A further include phenyl-(C₁-C₆)-alkanoyl, benzoylaminoacetyl, furoylaminoacetyl, β-(benzoylamino)acryloyl, *N*-(benzyloxycarbonyl)-histidinoyl, and naphthyl-acetyl.

The total length of the spacer (parts B and B 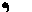 ) is preferably 6-12 atoms for PFT inhibitors and longer, up to 18 atoms, for PGGT-1 or ―2 inhibitors, the spacer may optionally contain multiple amide/amine linkages as well as other heteroatoms (e.g. S, O, etc.) and may be flexible or conformationally restricted. Preferably, B and/or B are residues of unnatural amino acids, such as β-, γ-, δ-, etc. aliphatic amino acids or amino acids containing a cyclohexylene or phenylene moiety between the amino group and the carboxyl group. The residue at the carboxy terminus (D) of the invented peptide analogues must contain one or more functionalities that can be deprotonated (i.e. negatively charged) under physiological conditions. The negatively charged groups are preferably carboxylic acids, phosphates, phosphonates, sulfonates and/or sulfates, most preferably carboxylates or phosphates. The terminal carboxy group of part D may be a carboxy group as such (or a metal salt thereof) or it may esterified (e.g. with an alcohol or a hdyroxyacid such as methanol, ethanol, glycolic or malic acid) or amidated or with peptide-like groups such as a single amino acid, possibly carrying a further negatively charged group (e.g. glycine, aspartic acid, glutamic acid, phosphoserine) or any oligopeptide, preferably a tetrapeptide containing a sugar amino acid derivative as described in EP-A-1028117.

Preferred peptide analogues according to the invention are analogues that are expected to be specific for the inhibition of farnesylation or geranylgeranylation processes as a function of their total length. It is anticipated that specific SS and GGPPS inhibitors will be obtained from the screening of a compound library due to the different mechanisms in which these enzymes convert FPP.

The peptide analogues according to the invention can be prepared in a manner which is known per se. For example, they may be prepared by starting with the suitably protected building block having the formula:

R²NH-(CH₂)₀₋₃-Z²-(CH₂)₀₋₃-CHR³-COOH

and coupling this unit to the terminal units :
Ap-(CH₂)₀₋₃-Z¹-(CH₂)₀₋₃-(CHR¹)₀₋₁COOH and NH₂-CH(COOX)-R⁴
Compounds in which Y is CH₂, rather than carbonyl, can be obtained by reduction or reductive amination, in a manner known per se. For example, these analogues may be prepared by starting with the suitably protected central unit having the formula:

R²NH-(CH₂)₀₋₃-Z²-(CH₂)₀₋₃-CHR³-CHO

And reacting this unit under reductive conditions with the terminal units:
Ap-(CH₂)₀₋₃-Z¹-(CH₂)₀₋₃-(CHR¹)₀₋₁CHO and NH₂-CH(COOX)-R⁴.

It was found that the peptide analogues according to the invention are capable of inhibiting the protein-farnesylation and/or geranylgeranylation process. Because of their ease of synthesis various inhibitors can be easily generated through combinatorial chemistry. The bioavailability, stability against enzymatic degradation and inhibitory activity of these novel inhibitors will be better than for previously reported compounds.

The peptide-like FPP-analogues described above are useful as an active substance in a pharmaceutical composition intended to interfere with protein prenylation or cholesterol biosynthesis. As such, they are useful as inhibitors in processes such as oncogenesis and other unwanted cell proliferation, e.g. in restenosis or atherosclerosis, and furthermore as suppressants of aberrant high signal transduction. Also, they can be used as anti-osteoporosis drugs and drugs to lower elevated plasma levels of cholesterol.

The pharmaceutical compositions containing the analogues according to the invention may be formulated in a usual way, e.g. by associating the inhibitors with a suitable solid or liquid carrier and optional adjuvants or other active components. The composition may be suitable for oral administration (capsule, pill, tablet, gel, powder, sachet, syrup, solution, dispersion etc.) or may be an injectable solution or another administration form. The composition may be administered to mammalians including man, in a dose which depends on the particular purpose of the administration and other conditions well known to the skilled person. A suitable dose is e.g. from 1 to 500 mg/kg body weight, especially from 10 to 200 mg/kg body weight. A dose can be administered in a single dosage or in several daily dosages.

The analogues of the invention can also be used in diagnostics procedures involving of assaying protein:prenyl-transferase activity in a biological sample or any other prenyl pyrophosphate consuming enzyme, by contacting the sample in which such activity is be determined with the peptide analogue and further reagents, enzyme substrates, labels (dyes, radioactive or fluorescent labels, antibodies etc.), diluents and the like as necessary for detecting the interaction of the peptide analogue with the enzyme under investigation.

### Example 1: Synthesis of protein:farnesyl and/or geranylgeranyl transferase inhibitors

6-Aminocaproic acid **1** (Aldrich) was treated with 9-fluorenylmethoxycarbonyl chloride (FmocCl) in dioxane with NaHCO₃ as a base, to produce compound **2** in 85 % yield after silica gel column chromatography (Scheme 1). Fmoc-Asp(O-*t*-Bu)-wang resin **3** (0.6 mmol/g, Novabiochem) was treated with 20 % piperidine in dimethylformamide (DMF) and subsequently coupled with compound **2** (5 equiv.) under a standard solid phase peptide synthesis protocol (Atherthon et al. *Solid Phase Synthesis: A Practical Approach*, IRL Press: Oxford, 1989) i.e. benzotriazole-1-yl-oxy-tris-(dimethylamino)phosphonium hexafluorophosphate (BOP), 1-hydroxybenzotriazole (HOBt), diisopropylethylamine (DiPEA) in *N*-methylpyrrolidone (NMP). The obtained compound **4** was subsequently treated with 20 % piperidine in DMF, coupled with 4-phenylbutyric acid (Aldrich) using the same reagents as mentioned above and cleaved from the solid support with trifluoroacetic acid (TFA)/triisopropylsilane 99.5/0.5 v/v %. The obtained compound **5** was purified using preparative HPLC (Lichrosphere 100 RP18 end-capped (5 µ), 10 x 250 mm, elution: linear gradient of 80 % acetonitrile/water/0.1 % TFA) and fully characterised with spectroscopic techniques.

### Example 2: Assays of protein:farnesyl transferase and protein:geranylgeranyl transferase 1

PFT activity was determined using a C-terminal peptide of pre-p21^{*N-ras*} coupled to sepharose beads as substrate (pepAsep) as described previously (Cohen et al. *Biochem. Pharmacol.* **57** (1999), 365-373). The experimental conditions (25 µL reaction mixture) were as follows: 80 pmol/25 µL sepharose-coupled peptide, 0.7 µM of [³H]-FPP (American Radiolabeled Chemicals Inc.; specific radioactivity 15 Ci/mmol) and 13 µL of rat brain enzyme preparation. Incubation was performed at 37°C for 30 min. For the determination of IC₅₀ values of the FPP analogues, the assay was performed three times in the presence of different concentrations of compound **5** (example 1) in duplicate.

Determination of PGGT-1 activity was performed in a similar manner as described for PFT activity and has also been described previously (Cohen et al. *Biochem. Pharmacol*. **57** (1999), 365-373). The same C-terminal peptide was used, except that the C-terminal methionine was replaced by leucine (pepCsep). The experimental conditions were as follows: 25 µL incubation mixture contained 2.5 µL (1 nmol) pepCsep, 1 µM [³H]-GGPP (American Radiolabeled Chemicals Inc.; specific radioactivity 15 Ci/mmol), 3 µL of bovine brain enzyme preparation, 50 µM ZnCl₂, 0.5 mM MgCl₂, 1 mM dithiothreitol, 0.004% Triton X-100, 50 mM Tris-HCl (pH 7.4). Incubation was performed at 37°C for 40 min. with continuous shaking. For the determination of IC₅₀ values of the GGPP analogues, the assay was performed three times in the presence of at least five different concentrations of the compound **5** in duplicate.

### Example 3:

According to the procedure as outlined in example 1, the following representative compounds have been prepared, with PFT IC₅₀ values being indicated. The compound with internal reference number TR 060 corresponds to compound **5** of examples 1 and 2.

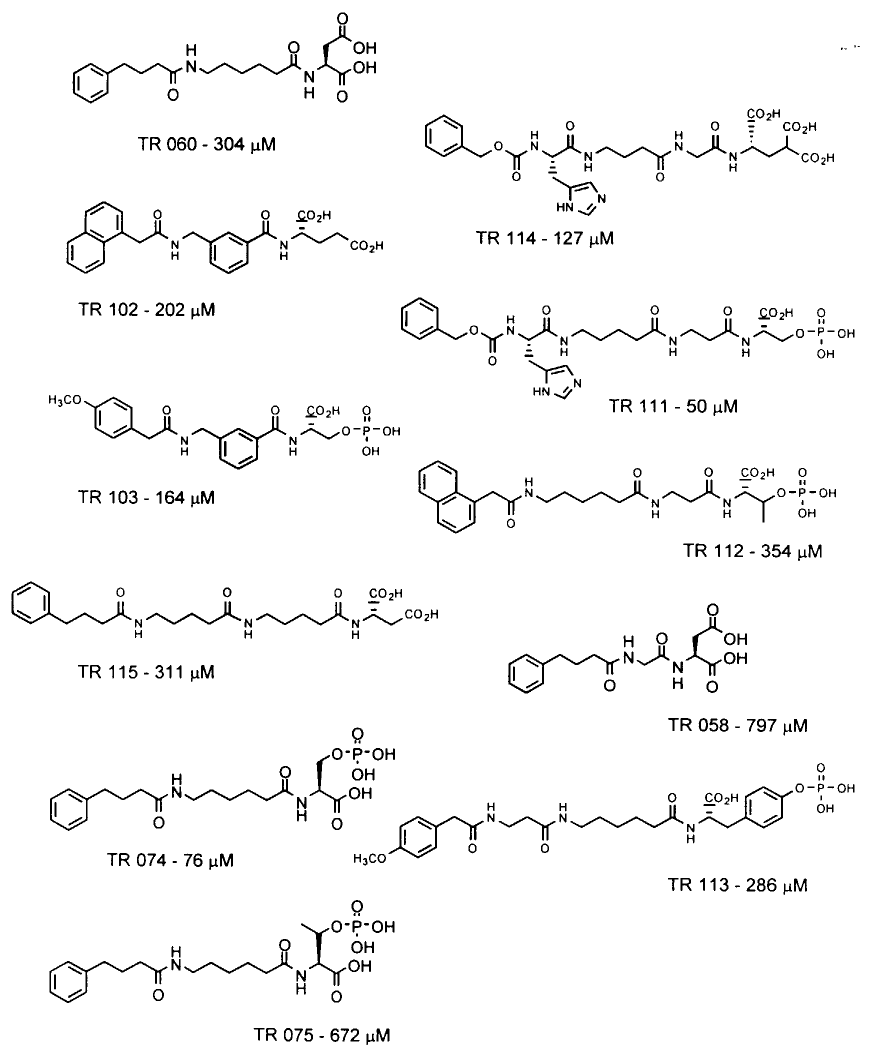

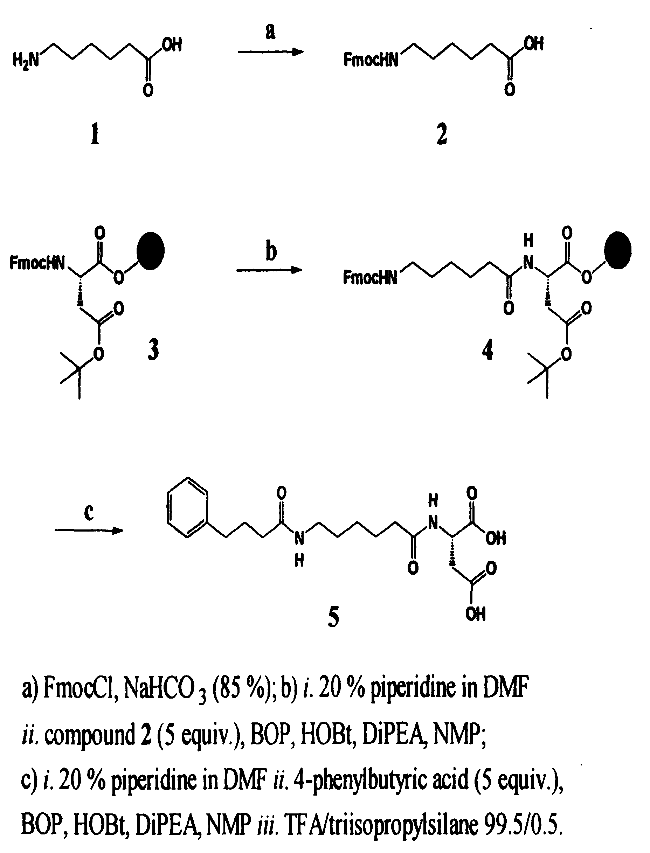

## Claims

1. A peptide analogue suitable as an inhibitor of a prenylpyrophosphate-consuming enzyme complying with formula A-B-D (1), or A-B-B'-D (2):
in which formulae:
A = Ap-(CH₂)₀₋₃-Z¹-(CH₂)₀₋₃-(CHR¹)₀₋₁-Y-
B and B' are independently -NR²-(CH₂)₀₋₃-Z²-(CH₂)₀₋₃-CHR³-Y-, or 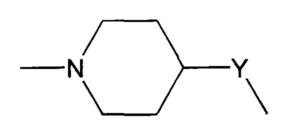
with the proviso that at least one of B and B' contains at least 4 chain atoms, i.e. if Z² is a direct bond, B and/or B' contains at least one CH₂ group;
D = -NH-CH(COX)-R⁴
in which:
Ap = an apolar hydrocarbon group containing at least 5 carbon atoms, optionally substituted with C₁₋₄ alkyl, C₁₋₄ alkoxy, halogen, methylenedioxy, aryl or aryloxy;
R¹ = hydrogen, or C₁₋₄ alkyl optionally substituted with aryl or heteroaryl;
R² = hydrogen, or C₁₋₄ alkyl optionally substituted with aryl or heteroaryl;
R³ = hydrogen, or C₁₋₄ alkyl optionally substituted with aryl, heteroaryl or amino;
R⁴ = C₁₋₄ alkyl, which is substituted with at least one group selected from carboxyl, phospho (PO₃H₂), phosphate (OPO₃H₂), sulpho (SO₃H), sulphate (OSO₃H), carboxyphenyl, sulphophenyl (C₆H₄SO₃H), phosphonophenyl (C₆H₄PO₃H₂) or phosphoxyphenyl (C₆H₄OPO₃H₂) groups;
X = OH, OM (M being an alkali metal), O-(C₁₋₄ alkyl optionally substituted with hydroxyl or carboxyl), NH-peptidyl or a group NH-CHR⁵-CT-NH-W-CT-NH-CH(COOR⁶)-C(R⁷R⁸)-R⁹, in which R⁵ is hydrogen, C₁₋₃ alkyl, mercaptomethyl or protected mercaptomethyl group, R⁶ is hydrogen or C₁₋₄ alkyl, R⁷ and R⁸ are independently hydrogen or C₁₋₄ alkyl and R⁹ is hydrogen or C₁₋₆ alkyl optionally substituted with hydroxy, phenyl, hydroxyphenyl, indolyl, imidazolyl, mercapto, methylthio, amino, carboxyl, carbamoyl, ureïdo, amidino or guanidino; T is oxo or two hydrogens, and W is a sugar amino acid analogue;
each Y = independently carbonyl or methylene;
Z¹ = -CH₂-CHR¹⁰-, -CH=CR¹⁰- (R¹⁰ is H or C₁₋₄ alkyl), -C≡C-, 1,2-, 1,3- or 1,4-cyclohexylene or -phenylene, -CO-, -CO-NH-, -O-CO-NH, or -O- or a direct bond;
Z² = -CH₂-CHR¹⁰-, -CH=CR¹⁰-, -C≡C-, 1,2-, 1,3- or 1,4-cyclohexylene or -phenylene, -O-, -S-, -NH-, or a direct bond;
or a salt or ester thereof.

2. A peptide analogue according to claim 1, in which said sugar amino acid analogue W has the formula: 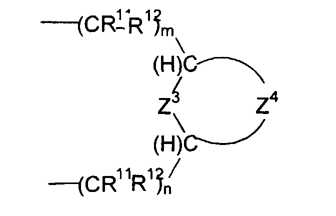 in which:
R¹¹ and R¹² are independently hydrogen or C₁₋₄ alkyl;
Z³ isoxygen, sulphur, imino or C₁₋₈ alkyl-, aryl- or acylimino;
Z⁴ is a direct bond or a saturated or unsaturated C₁₋₄ alkylene chain, optionally interrupted by one or more oxygen, sulphur or nitrogen atoms and optionally substituted with hydroxy, oxo, C₁₋₄ alkyl, C₁₋₄ alkylidene, C₁₋₄ alkoxy, allyloxy, benzyloxy, C₁₋₈ acyloxy, C₁₋₃ alkyl(id)enedioxy, amino, C₁₋₄ alkylamino or C₁₋₄ hydroxyalkyl;
(H) represents a hydrogen if Z⁴ is saturated at the corresponding C atom and is absent if Z⁴ is unsaturated at the corresponding C atom;
m is 0, 1 or 2;
n is 0 or 1.

3. A peptide analogue according to claim 1 or 2, complying with formula 1, in which one or more of the following definitions apply:
Ar is an optionally substituted aryl group, in particular phenyl, tolyl or methoxyphenyl;
R¹ is hydrogen, methyl or imidazolylmethyl;
at least one of R² and R³ is hydrogen;
at least one of Z¹ and Z² is a direct bond;
R⁴ is methyl, ethyl or benzyl substituted with one or two COOH or OPO₃H₂;
X is OH;
Y is carbonyl.

4. A peptide analogue according to any one of claims 1-3, in which A is:
Ar-(CH₂)₁₋₄-(O)₀₋₁-CO-, in particular benzoyl, phenylacetyl, benzyloxycarbonyl, 3-phenylpropanoyl, 4-phenylbutanoyl, or 2-(benzyloxycarbamoyl)-3-(2-imidazolyl)-propanoyl;

5. A peptide analogue according to any one of claims 1-4, in which B is:
-NH-(CH₂)₁₋₄-CH₂-CO- or -NH-(CH₂)₁₋₂-C₆H₄-(CH₂)₀₋₁-CO-, in particular 6-iminohexanoyl or 5-iminopentanoyl, 4-iminobutyryl or m-(iminomethyl)benzoyl.

6. A peptide analogue according to any one of claims 1-5, in which B' is:
-NH-(CH₂)₀₋₄-CHR³-CO- or B' is absent.

7. A peptide analogue according to any one of claims 1-36 in which D is the residue of phosphoserine, aspartate, glutamate or γ-carboxyglutamate.

8. Use of a peptide analogue according to any one of the preceding claims for preparing a medicament suitable for inhibiting protein:prenyl-transferases or any other prenyl pyrophosphate consuming enzyme e.g. for the use in treatment of osteoporosis, atherosclerosis, restenosis or cancer.

9. A pharmaceutical composition containing a peptide analogue according to any one of claims 1 - 7, together with a pharmaceutically acceptable carrier.

10. Use of a peptide analogue according to any one of claims 1-7 for assaying protein:prenyl-transferase activity in a biological sample or any other prenyl pyrophosphate consuming enzyme.
